# EUROPÄISCHE PATENTANMELDUNG

(11) **EP 2 452 615 A1**
(43) Veröffentlichungstag der Anmeldung: **16.05.2012**
(21) Anmeldenummer: 11188356.7
(22) Anmeldetag: 09.11.2011
(51) Int. Cl.: A61B 1/253, A61B 5/00, A61B 1/00, A61B 1/06

(54) **Zahnärztliches System zum Transilluminieren von Zähnen**

(30) Priorität: 11.11.2010 DE 102010043796
(71) Anmelder: Kaltenbach & Voigt GmbH, 88400 Biberach (DE)
(72) Erfinder: Hackel, André, 88400 Biberach (DE); Erdmann, Sven, 89081 Ulm (DE)
(74) Vertreter: Thun, Clemens

(57) **Zusammenfassung**

Bei einem zahnärztlichen System (1) zum Transilluminieren von Zähnen (100) mit einem länglichen Handstück (2) mit einer Lichtquelle zum Erzeugen einer Untersuchungsstrahlung, Bestrahlungsmitteln zum Richten der Untersuchungsstrahlung auf einen zu untersuchenden Zahn (100) sowie Mitteln zum Erfassen eines optischen Bildes des durch die Untersuchungsstrahlung illuminierten Zahns (100) ist am vorderen Ende des Handstücks (2) ein abnehmbarer Aufsatz (10, 110) angeordnet, der zumindest einen Teil der Bestrahlungsmittel beinhaltet, wobei das Handstück (2) Mittel zum Erkennen des an dem Handstück (2) angeordneten Aufsatzes (10, 110) aufweist.

## Beschreibung

Die vorliegende Erfindung betrifft ein zahnärztliches System zum Transilluminieren von Zähnen gemäß dem Oberbegriff des Anspruchs 1, welches ein längliches Handstück mit einer Lichtquelle zum Erzeugen einer Untersuchungsstrahlung, Bestrahlungsmittel zum Richten der Untersuchungsstrahlung auf einen zu untersuchenden Zahn sowie Mittel zum Erfassen eines optischen Bildes des durch die Untersuchungsstrahlung illuminierten Zahns aufweist, wobei am vorderen Ende des Handstücks ein abnehmbarer Aufsatz angeordnet ist, der zumindest einen Teil der Bestrahlungsmittel beinhaltet.

In der Medizin, insbesondere der Zahnmedizin werden vermehrt Diagnosesysteme eingesetzt, welche auf optischen Prinzipien beruhen. Grund hierfür ist, dass derartige Geräte üblicherweise berührungslos, also insbesondere schmerzfrei die Erstellung einer Diagnose ermöglichen und darüber hinaus auch oftmals optische Bilder zur Verfügung stellen, anhand welcher eventuell erforderliche therapeutische Maßnahmen dem Patienten anschaulich und damit besser vermittelt werden können. Beispielsweise kommen in der Zahnmedizin sogenannte Intraoralkameras zum Einsatz, welche ein Handstück beinhalten, dessen vorderer Endbereich in den Mund eines Patienten eingeführt wird. In diesem Endbereich befindet sich in der Regel ein Lichteintritts- bzw. Sichtfenster für die Kameraoptik, von dem ausgehend das Bild des zu untersuchenden Objekts einer Erfassungseinrichtung, beispielsweise einem CCD-Chip übermittelt wird.

Eine derartige Intraoralkamera kann ferner zu einem System zum Transilluminieren von Zähnen erweitert werden, wie es u. a. aus der DE 10 2006 041 020 A1 der Anmelderin bekannt ist. Hierbei wird der zu untersuchende Zahn mit Licht innerhalb eines bestimmten Wellenlängenbereichs bestrahlt, wobei dann ein optisches Bild des durch die Untersuchungsstrahlung illuminierten Zahns erfasst und bewertet wird. Da kariöse Stellen im Zahn das Licht anders streuen als gesundes Zahngewebe, können derartige Stellen bei Beobachtung des Zahns mit Hilfe einer Kamera identifiziert werden, wobei bei entsprechender Ausgestaltung des Systems sogar eine zuverlässigere Kariesdiagnose möglich ist als dies bei einer klassischen Röntgenuntersuchung der Fall ist.

Ein weiteres System zum Transilluminieren von Zähnen ist auch aus der DE 10 2009 013 615 A1 der Anmelderin bekannt. Insbesondere die Bestrahlungsmittel, mit deren Hilfe das Licht auf den zu untersuchenden Zahn gelenkt wird, sind hierbei in einem abnehmbaren Aufsatz angeordnet. Dies bringt zum einen Vorteile mit sich, da der Aufsatz getrennt von dem weiteren Handstück gereinigt und insbesondere desinfiziert werden kann. Auf der anderen Seite besteht - wie auch in der oben erwähnten DE 10 2009 013 615 A1 bereits beschrieben - die Möglichkeit, unterschiedliche Aufsätze zur Verfügung zu stellen, welche sich im Hinblick auf die Art und Weise, wie das Licht in den Zahn eingekoppelt und dieser dann beobachtet wird, unterscheiden. Es können dementsprechend unterschiedliche Zähne jeweils in optimaler Weise untersucht werden.

Ausgehend von diesem Stand der Technik liegt der vorliegenden Erfindung die Aufgabenstellung zugrunde, ein zahnärztliches System zum Transilluminieren von Zähnen zur Verfügung zu stellen, welches eine komfortablere Benutzung des Systems und damit letztendlich eine Optimierung der Untersuchung ermöglicht.

Die Aufgabe wird durch ein zahnärztliches System, welches die Merkmale des Anspruchs 1 aufweist, gelöst. Vorteilhafte Weiterbildungen der Erfindung sind Gegenstand der abhängigen Ansprüche.

Auch bei dem erfmdungsgemäßen zahnärztlichen System zum Transilluminieren von Zähnen ist vorgesehen, dass ein Handstück genutzt wird, an dessen vorderen Ende ein abnehmbarer Aufsatz angeordnet ist, der zumindest einen Teil der Bestrahlungsmittel beinhaltet. Erfindungsgemäß ist nunmehr vorgesehen, dass an dem Handstück Mittel vorhanden sind, mit deren Hilfe der an dem Handstück angeordnete Aufsatz automatisch erkannt wird.

Gemäß der vorliegenden Erfindung wird deshalb ein zahnärztliches System zum Transilluminieren von Zähnen vorgeschlagen, welches ein längliches Handstück mit einer Lichtquelle zum Erzeugen einer Untersuchungsstrahlung, Bestrahlungsmittel zum Richten der Untersuchungsstrahlung auf einen zu untersuchenden Zahn sowie Mittel zum Erfassen eines optischen Bildes des durch die Untersuchungsstrahlung illuminierten Zahns aufweist, wobei am vorderen Ende des Handstücks ein abnehmbarer Aufsatz angeordnet ist, der zumindest einen Teil der Bestrahlungsmittel beinhaltet, und wobei erfindungsgemäß das Handstück Mittel zum Erkennen des an dem Handstück angeordneten Aufsatzes aufweist. Vorzugsweise ist hierbei vorgesehen, dass verschiedene Aufsätze für das Handstück zur Verfügung stehen, wobei die Mittel zum Erkennen dazu ausgebildet sind, sowohl das Vorhandensein eines Aufsatzes als auch den Typ des aufgesteckten Aufsatzes zu erkennen.

Durch die Möglichkeit, das Vorhandensein eines Aufsatzes am vorderen Ende des Handstücks automatisch zu erkennen und ggf. sogar dessen Typ zu identifizieren, ergeben sich vielfältige Verbesserungen in der Handhabung des Systems. So kann insbesondere vorgesehen sein, dass dieses eine Steuerschaltung beinhaltet, welche in Abhängigkeit von Informationen, die von den Mitteln zum Erkennen des Aufsatzes zur Verfügung gestellt werden, die Lichtquelle zum Erzeugen der Untersuchungsstrahlung und/oder die Mittel zum Erfassen des optischen Bildes ansteuert. Beispielsweise könnte auf diesem Wege eine versehentliche Aktivierung der Lichtquelle bei nicht aufgesetztem Aufsatz verhindert werden. Auch eine Anpassung der Lichtstärke an den jeweils vorhandenen Aufsatz wäre denkbar, um beispielsweise eine Gefährdung durch zu hohe Bestrahlungsstärken zu vermeiden. In gleicher Weise wiederum könnten die Bilderfassungsmittel hinsichtlich ihrer Funktionsweise an den jeweils genutzten Aufsatz angepasst werden.

Stehen verschiedene Aufsätze zur Verfügung, so werden diese sich in erster Linie im Hinblick auf die Ausgestaltung der Bestrahlungsmittel unterscheiden, so dass unterschiedliche Möglichkeiten zur Verfügung stehen, Zähne zu beleuchten und zu beobachten. Denkbar wäre allerdings auch, dass zumindest einer der Aufsätze eine weitere Lichtquelle aufweist, mit deren Hilfe die Aufnahme eines insbesondere farbigen Reflexionsbilds einer dentalen Zahnoberfläche ermöglicht ist. Da beim Transilluminieren eines Zahns in der Regel eine verhältnismäßig schmalbandige Lichtquelle zum Einsatz kommt, sind mit derartigem Licht erstellte Reflexionsaufnahmen einer Zahnoberfläche wenig aussagekräftig. Durch die Nutzung einer zusätzlichen Lichtquelle, die beispielsweise durch eine Weißlichtquelle oder eine RGB-Lichtquelle realisiert werden kann, können nunmehr weitere optische Aufnahmen eines Zahns erstellt werden. Der Funktionsumfang des Systems kann auf diesem Wege derart erweitert werden, dass dieses auch als klassische Intraoralkamera genutzt werden kann. Der entsprechende Wechsel des Aufsatzes kann wiederum vorzugsweise durch das System selbstständig erkannt werden, was zu einem automatischen Betriebsartwechsel führt, bei dem entsprechende Parameter wie z. B. Blende, Fokus und/oder Brennweite der Bilderfassungsmittel angepasst werden.

Die Mittel zum Erkennen des Aufsatzes können in unterschiedlicher Weise ausgestaltet sein. Als bevorzugte Ausführungsformen haben sich Lichtschranken, insbesondere Gabellichtschranken, Mikroschalter oder Hallsensoren erwiesen.

Eine andere vorteilhafte Weiterbildung der vorliegenden Erfindung betrifft das Problem, dass ein feuchtes oder mit Speichel benetztes Objektfeld zu verstärkter Reflexion, Spiegelungen und/oder Brechungen führen kann, durch welche die Qualität des mit der Bildaufnahmeeinheit erfassten Bildes deutlich reduziert wird. Um dies zu vermeiden, ist gemäß einer vorteilhaften Weiterbildung der Erfindung eine Vorrichtung vorhanden, mit deren Hilfe ein Luftstrom auf ein Lichteintrittsfenster des Handstücks gerichtet wird. Dieser Luftstrom, der kontinuierlich oder bedarfsorientiert appliziert werden kann, bewirkt eine Trocknung des Objektfeldes, durch welche die oben beschriebenen Nachteile vermieden werden. Die Qualität der Aufnahmen und letztendlich die Zuverlässigkeit der Kariesdiagnose wird hierdurch deutlich erhöht.

Gemäß einer anderen vorteilhaften Weiterbildung der Erfindung sind ferner die Mittel zum Erfassen des optischen Bildes derart ausgestaltet, dass sie eine elektronische Bildaufnahmeeinheit sowie eine Bildauswerteeinheit umfassen. Hierbei kann insbesondere vorgesehen sein, dass die Bildauswerteeinheit die Bildaufnahmeeinheit derart ansteuert, dass Signale hoher Intensität abgeschwächt und Signale niedriger Intensität verstärkt werden. Durch diese Vorgehensweise können störende Effekte, die sich durch ungewünschte Reflexionen des Lichts ergeben, vermieden werden. Wiederum trägt dies zur Qualitätsverbesserung der Aufnahmen bei.

Nachfolgend soll die Erfindung anhand der beiliegenden Zeichnung näher erläutert werden. Es zeigen:
- Figur 1: die Ansicht eines erfindungsgemäßen zahnärztlichen Systems zum Transilluminieren von Zähnen;
- Figur 2: ein erstes Ausführungsbeispiel eines bei dem erfindungsgemäßen System zum Einsatz kommenden Aufsatzes;
- Figur 3: eine Schnittdarstellung des Aufsatzes von Figur 2;
- Figur 4: schematisch die Verwendung des Aufsatzes von Figur 2;
- Figur 5: ein zweites Ausführungsbeispiel eines bei dem erfindungsgemäßen System zum Einsatz kommenden Aufsatzes;
- Figur 6: eine Schnittdarstellung des Aufsatzes von Figur 5 und
- Figuren 7 bis 9: Darstellungen der Verwendung des zweiten Aufsatzes von Figur 5.

Bevor das erfindungsgemäße zahnärztliche Gerät detaillierter beschrieben wird, soll zunächst das der Untersuchungsmethode zugrunde liegende Verfahren kurz erläutert werden. Dieses Transilluminations-Verfahren beruht darauf, einen Zahn mit sichtbarem Licht zu durchleuchten bzw. zu transilluminieren. Hierzu wird mit Hilfe einer Lichtquelle eine Untersuchungsstrahlung generiert, welche auf den Zahn gerichtet wird. Die Untersuchungsstrahlung liegt dabei üblicherweise in einem Wellenlängenbereich von etwa 550 µm bis 790 µm, z. B. bei etwa 670 µm. Das Gewebe des Zahns blockt diese Untersuchungsstrahlung nicht vollständig ab, sondern erlaubt stattdessen, dass das Licht durch den Zahn hindurchtritt. Hierbei wird die Strahlung teilweise gestreut, wobei insbesondere kariöse Bereiche für eine charakteristische Beeinflussung des Lichts sorgen. Wird der auf diese Weise transilluminierte Zahn aus verschiedenen Blickrichtungen betrachtet, so können diese kariösen Bereiche erkannt werden, da diese etwas dunkler erscheinen. Insbesondere dann, wenn Aufnahmen, die zu verschiedenen Zeitpunkten erstellt wurden, miteinander verglichen werden, kann Karies auf diese Weise verhältnismäßig effektiv und auch frühzeitig erkannt werden.

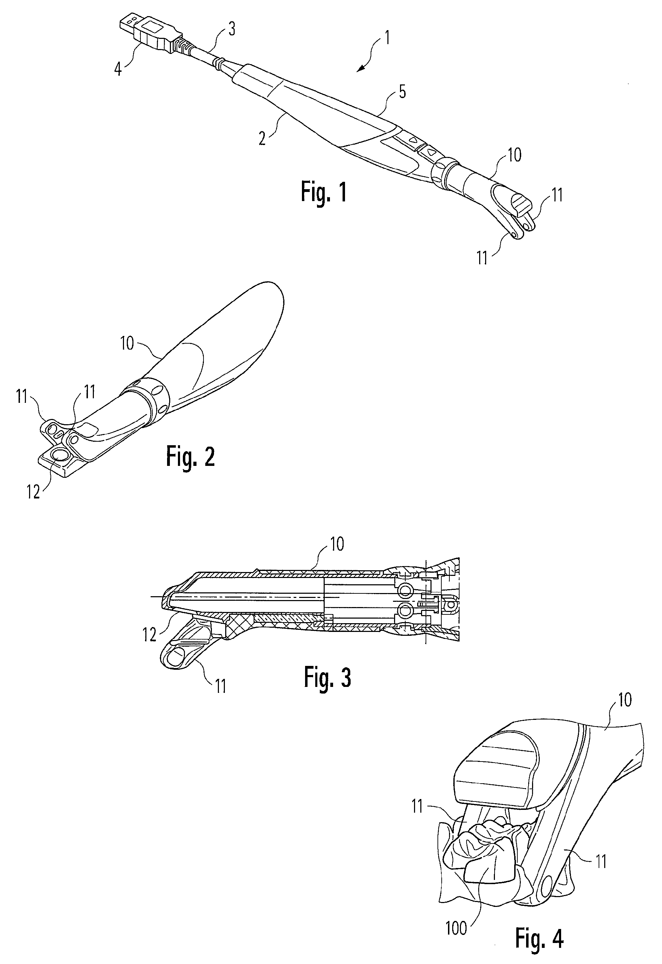

Ein auf diesem Untersuchungsprinzip beruhendes sogenanntes FOTI-(fibre-optik transillumination)-Gerät ist in Figur 1 dargestellt und allgemein mit dem Bezugszeichen 1 versehen. Wesentlicher Bestandteil ist ein handgehaltenes, längliches Instrument 2, das zum Beleuchten des zu untersuchenden Zahns genutzt wird und auch dazu dient, das optische Bild des illuminierten Zahns zu erfassen. Die hierbei gewonnenen Daten werden über ein Kabel 3, an dessen Ende sich ein USB-Stecker 4 befindet, an eine Zentraleinheit, insbesondere einen - nicht dargestellten - PC übermittelt. Hier erfolgt dann eine Darstellung der erstellten Bilder sowie eine Auswertung der Daten, um Karies zu erkennen. Selbstverständlich könnte die Verbindung mit der Zentrale auch anderweitig erfolgen, wobei der USB-Stecker 4 allerdings eine bevorzugte Ausführungsform darstellt, da über diesen gleichzeitig auch eine Stromversorgung des Geräts 1 erfolgt.

Das Handinstrument 2 besteht zunächst aus einer länglichen Griffhülse 5, in der die wesentlichen elektronischen Komponenten des Geräts 1 angeordnet sind. Es handelt sich hierbei einerseits um die entsprechende Elektronik zur Kommunikation über den USB-Anschluss mit der Zentrale sowie um Mittel zur Bilderfassung, Aufzeichnung und Bewertung. Hierbei kann es sich beispielsweise um einen CCD-Chip handeln, mit dessen Hilfe ein optisches Bild des Zahns aufgezeichnet wird.

Die optische Bildübertragung zu dem in der Griffhülse 5 befindlichen CCD-Chip erfolgt mit Hilfe eines Aufsatzes 10, der am vorderen Ende der Griffhülse 5 lösbar an dieser angeordnet ist. Dieser in den Figuren 2 bis 4 näher dargestellte Aufsatz 10 dient einerseits zum Beleuchten des zu untersuchenden Zahns 100, wie dies in Figur 4 dargestellt ist. Hierzu dienen zwei seitliche Arme 11, durch welche sich Lichtleiter erstrecken. An den beiden Enden der Arme 11 wird das Licht das seitlich umgelenkt und in den Zahn 100 eingekoppelt. Zum anderen wird mit Hilfe des Aufsatzes 10 ein Bild des illuminierten Zahns 100 erfasst und an die Bilderfassungsmittel, also beispielweise den CCD-Chip auf optischem Wege weitergeleitet. Der Aufsatz 10 weist hierzu an seinem vorderen Ende ein zur Unterseite hin gerichtetes Fenster 12 auf, welches dann über weitere optische Elemente mit den Bilderfassungsmitteln gekoppelt ist. Zur Bildweiterleitung werden in erster Linie Spiegel, Prismen, Linsen und dergleichen verwendet.

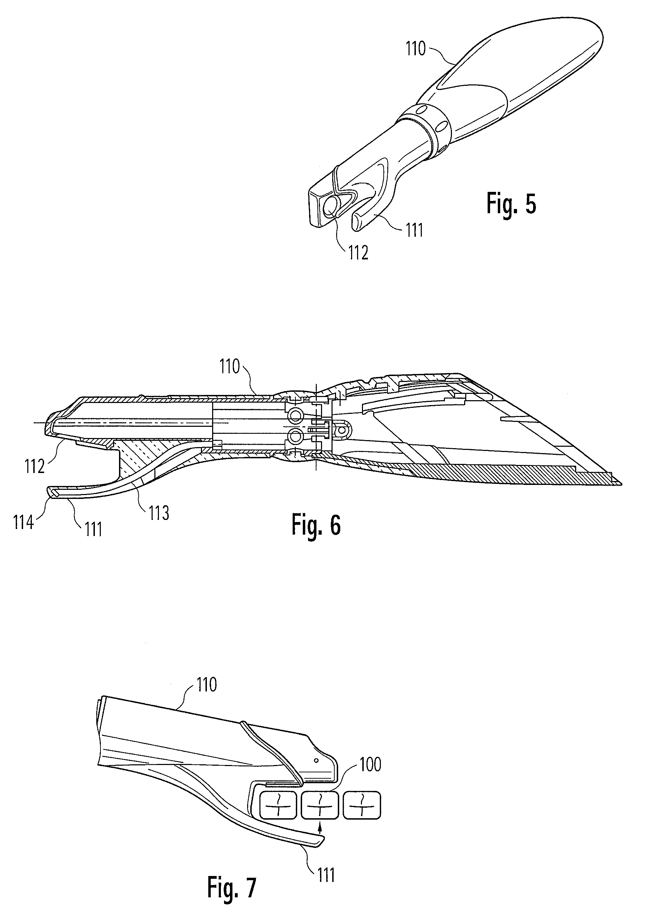

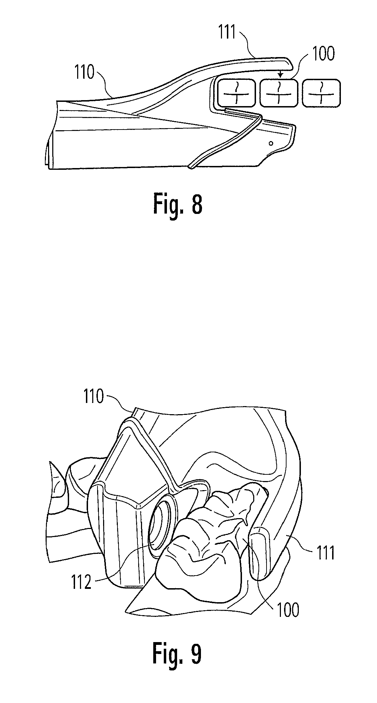

Die Figuren 5-9 zeigen einen weiteren Aufsatz 110, der bei dem erfindungsgemäßen Gerät 1 zur Kariesdiagnose ebenfalls zum Einsatz kommen könnte. Im Unterschied zu dem zuvor diskutierten Aufsatz 10 weist dieser weitere Aufsatz 110 lediglich einen einzigen Arm 111 auf, durch den sich ein Lichtleiter 113 mit einem am vorderen Ende befindlichen Umlenkelement 114 erstreckt. Diesem vorderen Ende des Arms 111 gegenüberliegend ist wiederum der Endbereich mit einem Lichteintrittsfenster 112 angeordnet. Im aufgesetzten Zustand des Aufsatzes 110 auf der Griffhülse 5 des Handstücks 2 befindet sich unmittelbar hinter dem Eintrittsfenster 112 ein Umlenkprisma, mit dessen Hilfe das von dem zu untersuchenden Zahn einfallende Licht auf die Bilderfassungsmittel gerichtet wird.

Die einander gegenüberliegende Anordnung von Umlenkelement 114 und Lichteintrittsfenster 112 hat zur Folge, dass mit Hilfe dieses Aufsatzes 110 der zu untersuchende Zahn 100 wahlweise von der sogenannten bukalen Zahnfläche beleuchtet und der lingualen Zahnfläche beobachtet werden kann oder alternativ hierzu auch von der lingualen Zahnfläche beleuchtet und von der bukalen Zahnfläche aus beobachtet werden kann. Im Vergleich zu bislang üblichen Ausführungsformen ist bei einem derartigen Wechsel kein Austausch des Aufsatzes erforderlich, was eine wesentliche Arbeitserleichterung und Zeitersparnis darstellt. Ferner muss das Handstück auch nicht aus dem Arbeitsraum, also dem Mundraum des Patienten herausgenommen werden und zum Wechsel der Betrachtungsrichtung ist keine Zweihandbedienung erforderlich. Der Aufsatz 110 ist hierbei derart auf dem Handstück 2 angeordnet, dass er nicht verdreht werden kann. Hierdurch wird eine ergonomische Handhabung erzielt.

Bei dem erfindungsgemäßen System kommen also zumindest zwei unterschiedliche Aufsätze 10, 110 zum Einsatz, welche für den jeweiligen Anwendungsfall eine optimale Untersuchung eines Zahns ermöglichen. Aufgrund der unterschiedlichen Ausgestaltungen der Aufsätze 10, 110 kann es allerdings erforderlich sein, die Lichtabgabe sowie die Sensitivität der Bilderfassungsmittel in geeigneter Weise anzupassen. So könnte beispielsweise für den zweiten Aufsatz 110 eine höhere Leuchtstärke erforderlich sein, da hier die Beleuchtung des zu untersuchenden Zahns lediglich von einer Seite aus erfolgt.

Um trotz allem die Handhabung des erfindungsgemäßen Systems einfach und komfortabel zu gestalten, ist vorgesehen, dass der aufgesetzte Aufsatz 10 bzw. 110 automatisch erkannt wird. Das heißt, an der Griffhülse 5 des Instruments 2 sind Mittel vorgesehen, über welche erkannt wird, ob sich ein Aufsatz an der Vorderseite der Griffhülse 5 befindet und - falls ja - um welchen Aufsatztyp es sich handelt. Derartige Mittel können insbesondere in Form einer Gabellichtschranke ausgeführt sein, wobei in diesem Fall bei Aufstecken eines Aufsatzes ein Teil des Aufsatzgehäuses bzw. ein entsprechende Vorsprung in die an der Griffhülse befindliche Gabellichtschranke eintaucht. Dieser Teil des Aufsatzes kann opak, farbig, transluzent oder optisch undurchlässig ausgeführt sein, wobei dann durch eine jeweils spezifische Ausgestaltung dieses Bereichs eine Identifizierung des Aufsatztyps ermöglicht ist. Alternativ zu dieser Ausführungsform könnte eine automatische Erkennung des Aufsatzes auch mit Hilfe von Mikroschaltern oder Hallsensoren mit entsprechenden Magneten ausgeführt sein.

Intern weist dann das erfindungsgemäße Gerät 2 eine Steuereinheit auf, welche auf Basis der von den Mitteln zum Erkennen des Aufsatzes zur Verfügung gestellten Informationen eine entsprechende Ansteuerung der verschiedenen Komponenten übernimmt. Im einfachsten Fall kann hierzu vorgesehen sein, dass lediglich bei aufgestecktem Aufsatz eine Aktivierung der Lichtquelle(n) ermöglicht wird, während hingegen für den Fall, dass kein Aufsatz erkannt wurde, die Lichtquelle(n) und ggf. erweiterte Funktionseinheiten der Elektronik deaktiviert werden, um z. B. eine Gefährdung durch zu hohe Bestrahlungsstärken zu vermeiden. Auch eine Anpassung der Leuchtstärke an den Aufsatztyp kann - wie oben bereits erwähnt - hierdurch vorgenommen werden. Das Gerät erkennt also selbstständig, welcher Aufsatz auf die Griffhülse aufgesetzt wurde und übernimmt eine entsprechend geeignete Ansteuerung der weiteren Komponenten des Systems, sodass diese Einstellungen nicht manuell durch einen Benutzer vorzunehmen sind. Die Handhabung des Geräts gestaltet sich dementsprechend trotz der unterschiedlichen Möglichkeiten äußerst einfach. Anzumerken ist ferner, dass die oben genannte Gefährdung durch zu hohe Bestrahlungsstärken auch dadurch vermieden werden könnte, dass ein Bedienelement vorgesehen ist, welches die Abnahme des Aufsatzes bei aktivierter Lichtquelle verhindert. Ferner wäre die Nutzung zur Erkennung des Vorhandenseins eines Aufsatzes auch dann sinnvoll, wenn lediglich ein einziger Aufsatz zur Verfügung stehe, da auch in diesem Fall eine Fehlbedienung, insbesondere eine Aktivierung der Lichtquelle(n) bei nicht vorhandenem Aufsatz ausgeschlossen werden sollte.

Eine weitere vorteilhafte Weiterentwicklung des erfindungsgemäßen Systems beschäftigt sich mit der Auswertung der von dem CCD-Chip erhaltenen Bildinformationen. In diesem Zusammenhang ist zu berücksichtigen, dass Licht, welches direkt oder per Reflexion von der Beleuchtungseinheit in die Bildaufnahmeeinheit, also beispielsweise den CCD-Chip gelangt, ohne das humane Gewebe zu durchqueren, eine deutlich höhere Intensität aufweist, als das Licht, welches zunächst den Zahn durchdringt und hier durch teilweise Absorption abgeschwächt wird. Durch eine geeignete Ausführung der erforderlichen Bildauswerteeinheit können nunmehr die räumlichen Bereiche der Bildaufnahmeeinheit, welche mit einer deutlich höheren Intensität belichtet werden, detektiert werden. Dies kann beispielsweise im Rahmen einer sogenannten Histogrammanalyse erfolgen. Die Bildauswerteeinheit kann dann die Bildaufnahmeeinheit derart ansteuern, dass der zugehörige Helligkeitswert der überstrahlten Bereiche abgeschwächt wird, während hingegen gleichzeitig diejenigen Bereiche der Bildaufnahmeeinheit, welche eine deutlich geringere Lichtintensität aufweisen, verstärkt werden, so dass das zugehörige Helligkeitssignal angehoben wird. Die auf diese Weise in ihrem Helligkeitswert modifizierten Bereiche der Bildaufnahmeeinheit werden dann zusammen in einem Bild mit beschränktem Helligkeitsumfang (beispielsweise 256 Graustufen) dargestellt, wodurch letztendlich ein Bild mit einem deutlich erhöhten Dynamikbereich erhalten wird. Mit anderen Worten, die Qualität der dargestellten optischen Aufnahmen, welche letztendlich für die Zuverlässigkeit der Kariesdiagnose ausschlaggebend ist, wird auf diesem Wege deutlich verbessert.

Eine weitere Maßnahme zur Verbesserung der Bildqualität besteht in der Realisierung eines sterilisierbaren Aufsatzes mit einem integrierten Luftkanal. Hintergrund dieser Verbesserung ist, dass ein feuchtes oder mit Speichel benetztes Objektfeld zu verstärkter Reflexion, zu Spiegelungen oder Brechungen an Luftblasen, die sich durch Speichel bilden, führt. Derartige Effekte führen zwangsläufig zu einer verminderten Qualität des erfassten Bildes.

Um derartige Effekte zu vermeiden, wird nunmehr gemäß einer bevorzugten Ausführungsform vorgeschlagen, auf das Lichteintrittsfenster eines Aufsatzes einen Luftstrom zu richten. Durch diesen Luftstrom kann eine Beschlagfreiheit der optischen Elemente zu Bildaufnahmen sowie eine Trocknung des Objektfeldes erzielt werden. Vorzugsweise wird hierbei die Luft über den aufsteckbaren Aufsatz auf das optische Element und ggf. auch auf den zu untersuchenden Zahn gerichtet. Hierbei kann der Luftstrom kontinuierlich appliziert werden. Denkbar wäre allerdings auch eine bedarfsorientierte manuelle Zuschaltung durch den Benutzer des Geräts. Auch eine automatische Zu- und Abschaltung in Verbindung mit einem Sensorelement, welches das Vorhandensein von Feuchte auf dem aufzunehmenden Objektfeld detektiert, wäre denkbar.

Wesentlich hierbei ist, dass der Luftkanal zumindest in seinem letzten Abschnitt durch den Aufsatz selbst hindurchgeführt wird. Hierdurch besteht die Möglichkeit, im Rahmen der Sterilisation des Aufsatzes auch diesen Kanal keimfrei zu halten. Der in den Aufsatz integrierte Luftkanal steht dann mit einer sich durch die Griffhülse des Handstücks erstreckenden Zuführungsleitung für die Luft in Verbindung.

Die beiden dargestellten Aufsatz-Varianten 10 und 110 dienen beide dazu, Transilluminations-Aufnahmen von Zähnen zu erhalten. Denkbar wäre allerdings auch, einen weiteren Aufsatz zur Verfügung zu stellen, der eine zusätzliche Lichtquelle beinhaltet, welche die Aufnahme eines farbigen Reflexionsbilds einer dentalen Zahnoberfläche ermöglicht. Hierfür sind die in den Figuren dargestellten Aufsätze eher weniger gut geeignet, da diese lediglich Licht mit einer sehr schmalen spektralen Bandweite emittieren. Für die Diagnose von Karies im Rahmen der Transillumination ist dies von Vorteil, allerdings können dann keine klassischen optischen Aufnahmen einer Zahnoberfläche erstellt werden.

Die nunmehr vorgestellte Erweiterung der Erfindung sieht dementsprechend vor, dass ein weiterer Aufsatz eine zusätzliche Lichtquelle, beispielsweise eine Weißlicht-Lichtquelle oder eine RGB-Lichtquelle aufweist. Diese ist in Bezug auf das entsprechende Lichteintrittsfenster des Aufsatzes derart ausgestaltet und angeordnet, dass sie den von dem Lichtaustrittsfenster erfassten Bereich unmittelbar beleuchtet und dementsprechend die Erstellung eines klassischen Reflexionsbildes gestattet. Letztendlich kann mit Hilfe dieses zusätzlichen Aufsatzes das System also auch als klassische Intraoralkamera genutzt werden. Für den Fall, dass eine RGB-Lichtquelle zum Einsatz kommt, kann das Farbbild auch aus mehreren Einzelbildern in einer nachgeschalteten Bildauswerteeinrichtung generiert werden. In diesem Fall werden also die einzelnen Farben aufeinanderfolgend aktiviert, wobei dann die jeweils erhaltenen Bilder überlagert und zu der Gesamtaufnahme zusammengestellt werden. Der hierzu verwendete Aufsatz weist dann entsprechende Anschlüsse auf, über welche einerseits eine Stromversorgung der Lichtquelle bzw. Lichtquellen ermöglicht wird und andererseits die Ansteuerung der Lichtquelle(n) vorgenommen wird. Wiederum kann vorgesehen sein, dass das Aufsetzen dieses zusätzlichen Aufsatzes automatisch durch das Gerät erkannt und dementsprechend ein Wechsel in eine andere Betriebsart initiiert wird. Bei diesem Betriebsartwechsel können dann bestimmte Parameter der Mittel zur Bildaufnahme, beispielsweise Blende, Fokus und/oder Brennweite entsprechend angepasst werden.

Schließlich besteht eine weitere vorteilhafte Weiterbildung der Erfindung auch darin, dass die Mittel zur Bildauswertung eine kontinuierliche Aufnahme ermöglichen, was letztendlich das Erstellen einer sogenannten Panoramaansicht gestattet. In diesem Fall wird mit dem Gerät der gesamte Zahnbogen abgefahren, wobei hierbei kontinuierlich Bilddaten bzw. Messdaten erfasst werden. Zur Komprimierung der Messdatenaufnahme wäre es denkbar, die zeitlich versetzt aufgenommenen Daten einzelner Teile des Zahnbogens in einer Übersicht zusammenzufassen. Beispielsweise kann durch das Zusammenfügen von Einzelbildern eines Live-Videostreams ein Einzelbild eines größeren Teils des Zahnbogens bzw. auch des kompletten Zahnbogens erzeugt werden. Derartige Panoramaansichten, die bislang ausschließlich mit entsprechend gestalteten Röntgengeräten erstellt werden konnten, erlauben es, den Patienten in besonders anschaulicher und übersichtlicher Weise erforderliche therapeutische Maßnahmen zu erläutern.

Letztendlich wird also ein System zum Transilluminieren von Zähnen geschaffen, welches für einen Benutzer in besonders einfacher und komfortabler Weise zu handhaben ist. Gleichzeitig gestattet es allerdings auch, Aufnahmen in sehr hoher Qualität zu erstellen. Letztendlich wird hierdurch die Möglichkeit zur frühzeitigen Erkennung von Karies deutlich verbessert.

## Patentansprüche

1. Zahnärztliches System (1) zum Transilluminieren von Zähnen (100), aufweisend:
• ein längliches Handstück (2) mit einer Lichtquelle zum Erzeugen einer Untersuchungsstrahlung,
• Bestrahlungsmittel zum Richten der Untersuchungsstrahlung auf einen zu untersuchenden Zahn (100),
• sowie Mittel zum Erfassen eines optischen Bildes des durch die Untersuchungsstrahlung illuminierten Zahns (100),
wobei am vorderen Ende des Handstücks (2) ein abnehmbarer Aufsatz (10,110) angeordnet ist, der zumindest einen Teil der Bestrahlungsmittel beinhaltet,
**dadurch gekennzeichnet,**
**dass** das Handstück (2) Mittel zum Erkennen des an dem Handstück (2) angeordneten Aufsatzes (10, 110) aufweist.

2. Zahnärztliches System nach Anspruch 1,
**dadurch gekennzeichnet,**
**dass** verschiedene Aufsätze (10, 110) für das Handstück (2) zur Verfügung stehen, wobei die Mittel zum Erkennen dazu ausgebildet sind, sowohl das Vorhandensein eines Aufsatzes (10, 110) als auch den Typ des Aufsatzes (10, 110) zu erkennen.

3. Zahnärztliches System nach Anspruch 2,
**dadurch gekennzeichnet,**
**dass** sich die Aufsätze (10, 110) im Hinblick auf die Ausgestaltung der Bestrahlungsmittel unterscheiden.

4. Zahnärztliches System nach Anspruch 2 oder 3,
**dadurch gekennzeichnet,**
**dass** zumindest einer der Aufsätze eine zusätzliche Lichtquelle aufweist.

5. Zahnärztliches System nach Anspruch 4,
**dadurch gekennzeichnet,**
**dass** es sich bei der zusätzliche Lichtquelle um eine Weißlichtquelle oder eine RGB-Lichtquelle handelt.

6. Zahnärztliches System nach einem der vorherigen Ansprüche,
**dadurch gekennzeichnet,**
**dass** die Mittel zum Erkennen des Aufsatzes (10, 110) eine Lichtschranke, Mikroschalter oder Hallsensoren aufweisen.

7. Zahnärztliches System nach einem der vorherigen Ansprüche,
**dadurch gekennzeichnet,**
**dass** dieses eine Steuerschaltung beinhaltet, welches in Abhängigkeit von Informationen, die von den Mitteln zum Erkennen des Aufsatzes (10, 110) zur Verfügung gestellt werden, die Lichtquelle zum Erzeugen der Untersuchungsstrahlung und/oder die Mittel zum Erfassen des optischen Bildes ansteuert.

8. Zahnärztliches System nach einem der vorherigen Ansprüche,
**dadurch gekennzeichnet,**
**dass** die Mittel zum Erfassen des optischen Bildes ein sich am vorderen Ende des Handstücks (2) befmdendes Lichteintrittsfenster (12, 112) umfassen,
wobei das System eine Vorrichtung zum Richten eines Luftstroms auf das Lichteintrittsfenster (12, 112) aufweist.

9. Zahnärztliches System nach einem der vorherigen Ansprüche,
**dadurch gekennzeichnet,**
**dass** die Vorrichtung zum Richten des Luftstroms einen sich durch den Aufsatz (10, 110) erstreckenden Luftkanal aufweist.

10. Zahnärztliches System nach einem der vorherigen Ansprüche,
**dadurch gekennzeichnet,**
**dass** die Mittel zum Erfassen des optischen Bildes eine elektronische Bildaufnahmeeinheit sowie eine Bildauswerteeinheit umfassen, wobei die Bildaufnahmeeinheit dazu ausgebildet ist, die Bildauswerteeinheit derart anzusteuern, dass Signale hoher Intensität abgeschwächt und Signale niedriger Intensität verstärkt werden.
